(19) Europäisches Patentamt
European Patent Office
Office européen des brevets

(11) Veröffentlichungsnummer: **0 327 920**
**A2**

(12) # EUROPÄISCHE PATENTANMELDUNG

(21) Anmeldenummer: **89101587.7**

(51) Int. Cl.⁴: **C07H 19/01**

(22) Anmeldetag: **30.01.89**

(30) Priorität: **04.02.88 DE 3803339**

(43) Veröffentlichungstag der Anmeldung:
**16.08.89 Patentblatt 89/33**

(84) Benannte Vertragsstaaten:
**AT BE CH DE FR GB IT LI NL SE**

(71) Anmelder: **SÜDZUCKER AKTIENGESELLSCHAFT MANNHEIM/OCHSENFURT**
**Maximilianstrasse 10**
**D-6800 Mannheim 1(DE)**

(72) Erfinder: **Gander, Michael**
**Odenwaldstrasse 3**
**D-6520 Worms 21(DE)**
Erfinder: **Rapp, Knut M., Dr.rer.nat.**
**Im Kerner 16**
**D-6521 Offstein(DE)**
Erfinder: **Schiweck, Hubert, Dr.**
**John-F.-Kennedy-Strasse 5**
**D-6520 Worms(DE)**

(74) Vertreter: **Körber, Wolfhart, Dr. et al**
**Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10**
**D-8000 München 22(DE)**

(54) **Verfahren zur Herstellung von 1,6-beta-D-Anhydroglucopyranose (Levoglucosan) in hoher Reinheit.**

(57) Die Erfindung betrifft ein Verfahren zur Herstellung von 1,6-β-D-Anhydroglucopyranose (Levoglucosan) aus Stärke und/oder stärkehaltigem bzw. cellulosehaltigem Material durch Vakuumpyrolyse.

Für eine groß-technische Herstellung von Levoglucosan in hoher Reinheit sind die bekannten Verfahren zu umständlich und/oder zu teuer. Bei der bekannten Stärkepyrolyse wird eine Vielzahl von Nebenprodukten gebildet, so daß langwierige Reinigungsverfahren unter Verwendung von organischen Lösungsmitteln und langsame Kristallisation üblich sind. Niedrige Ausbeuten und mangelnde Reinheit charakterisieren die bekannten Levoglucosan-Produkte.

Es wird ein Verfahren zur Herstellung von Levoglucosan zur Verfügung gestellt, bei dem das Ausgangsmaterial mit einem auf max. 20 % eingestellten Wassergehalt depolymerisiert wird, die flüchtigen Bestandteile kondensiert werden und das Kondensat nach Neutralisation, Filtration und ggf. Behandlung mit einem Adsorptionsmittel, einer chromatographischen Trennung über Ionenaustauscher, mit Wasser als Elutionsmittel, unterworfen und die Levoglucosan enthaltenden Fraktionen eingeengt und durch eine Verdampfungs- und/oder Kühlungskristallisation aus Wasser kristallines Levoglucosan erhalten wird.

EP 0 327 920 A2

## Verfahren zur Herstellung von1,6-β-D-Anhydroglucopyranose (Levoglucosan) in hoher Reinheit

Chemierohstoffe werden heute zum größten Teil aus Erdöl gewonnen. Die Endlichkeit fossiler Vorräte und das Problem der Verwertung von landwirtschaftlichen Überschußprodukten in der EG und den USA fordern eine Verwendung von "Nachwachsenden Rohstoffen" für Produkte, die bisher aus Erdöl hergestellt werden.

Als Kohlenhydrate kommen neben Saccharose insbesonders Stärke in Frage, da beispielsweise Getreide (70 % Stärke) jährlich in vergleichbaren Mengen (~ $10^9$ t) produziert wie Erdöl gefördert wird.

Chemierohstoffe müssen heute in großen Mengen und zu akzeptablen Preisen verfügbar sein. In besonderem Maße müssen sie die Kriterien der einfachen, sicheren und umweltfreundlichen Produktion, der vielseitigen Modifizierbarkeit und Verwendungsfähigkeit und der Umweltverträglichkeit erfüllen.

Ein in einfachen Schritten herstellbares Produkt, das die oben geforderten Kriterien erfüllt, ist 1,6-β-D-Anhydroglucopyranose (Levoglucosan) mit der folgenden Strukturformel

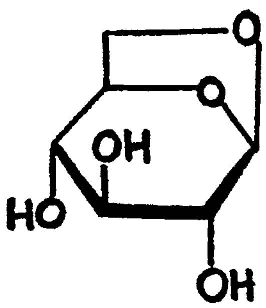

(I)

Levoglucosan ist also ein Derivat der Glucose, des häufigsten Zuckers auf der Erde.

Die Chemie von 1,6-β-D-Anhydroglucopyranose (Levoglucosan) ist lange bekannt, und seine vielseitigen Reaktionsmöglichkeiten sind gut untersucht (M. Černý und J. Staněk, Adv. Carbohydr. Chem. Biochem., 34, 23 (1977) Academic Press, New York, San Francisco, London); eine größere industrielle Verwertung diese Produktes scheiterte jedoch bisher an einer einfachen Herstellung in größerem Maßstab.

Das synthetische Potential von Levoglucosan erstreckt sich über sehr vielseitige Einsatzgebiete, wie die Synthese von Oligosacchariden oder von (+)-Biotin (Carbohydr. Res. 57, C31-C35 (1977), und die Möglichkeit, daraus Pharmaka, z.B. Cytostatika (DE-OS 34 24 217), oder Herbizide bzw. pflanzenwuchsregulierende Wirkstoffe (EU 0 229 034) herzustellen.

Die Chiralität von Levoglucosan gestattet es, den Phosphinliganden DIOXOP daraus zu synthetisieren, der Bestandteil eines Katalysators für die diastereoselektive Hydrierung ist (M. Nógrádi, Stereoselective Synthesis, VCH Verlagsgesellschaft mbH, Weinheim, S. 65,79 (1987)), oder andere chirale 2,4-disubstituierte 1,3-Dioxolane herzustellen.

Die sterische Spannung des Anhydrozuckers erlaubt es, Derivate z. B. Trialkylether mit geeigneten Katalysatoren zu 1,6-verknüpften Polymeren umzusetzen (Polymer J. Vol. 16, No. 3 pp 297-301 (1984); Vysokomol. Soedin., Ser. A 1984, 26 (10), 2173-80, zit. in Chem. Abstr. 102, 95 916q); auch werden Derivate des Levoglucosans für die dreidimensionale Polymerisation verwendet (SU 862 567, zit. in Chem. Abstr. 101, 73 215 n; SU 1 038 344, zit. in Chem. Abstr. 100, 175 205 f).

Für die Herstellung von Levoglucosan sind unterschiedliche Wege bekannt, beispielsweise:

a) die Pyrolyse von Stärke (Methods Carbohydr. Chem. 2, 394 (1963)),

b) die Umsetzung von Phenyl-2,3,4,6-tetra-O-acetyl-β-D-glucopyranosid mit Alkali (Methods Carbohydr. Chem. 2, 397 (1963)),

c) die Umsetzung von Pentachlorphenyl-2,3,4,6-tetra-O-acetyl-β-D-glucopyranosid mit Tetrabutylammoniumhydroxid (Carbohydr. Res. 101, 148 (1982)),

d) die Pyrolyse von säurebehandeltem (z. B. mit Monochloressigsäure) Hartholz mit überhitztem Dampf (SU 1 133 279, zit. in Chem. Abstr. 102, 204 235 q, (1985)),

e) die basenkatalysierte Fluorwasserstoffabspaltung aus α-D-Glucopyranosylfluorid (vgl. Phytochemistry 21, 2301 (1982)),

f) die Pyrolyse von vorbehandelter Stärke (US 3 478 012) und

g) die Pyrolyse von mit Säuren und Basen vorbehandelter Cellulose mit überhitztem Dampf, vorzugsweise zwischen 350° und 600° C, und Aufarbeitung mittels organischer Lösungsmittel (US 3 235 541).

Als weitere Methoden sind noch zu nennen:

h) In einer neueren Synthese für Levoglucosantriacetat (Kohlenhydrate werden häufig aus Gründen der leichteren Isolierung in acetylierter Form dargestellt) wird 1,2,3,4,-Tetra-O-acetyl-6-O-triphenylmethyl-β-D-glucopyranose mit verschiedenen Lewis-Säuren umgesetzt, wobei teilweise hohe Ausbeuten an Levoglucosantriacetat erhalten werden (Carbohydr. Res. 162, 141 (1987)).

i) Reinigungsverfahren levoglucosanhaltiger Gemische werden z. B. in Mater. Nauch.-Tekh. Konf. Khim.-Tekhnol. Fak., Leningrad. Lesotekh. Akad. 1967, 90, zit. in Chem. Abstr. 71, 82 868s beschrieben. Eine Entsalzung über saure und basische Ionenaustauscher führt zwar zu einer Entfärbung der Rohlösung, der Anionenaustauscher verliert jedoch seine Wirksamkeit ziemlich rasch. Eine alternative Methode, die fraktionierte Fällung von Levoglucosan aus butanolischer Lösung mit Diethylether erfordert den Einsatz teurer und gefährlicher organischer Lösungsmittel.

k) In US 3 374 222 wird eine levoglucosanhaltige Rohlösung, die aus Holz und lignocellulosehaltigem Material über eine Pyrolyse erhalten wird, auf einen pH-Wert von 12 oder höher gebracht. Durch mehrere Schritte wie Kühlen, Verdünnen, Erhitzen, Filtrieren, erhält man schließlich eine levoglucosanhaltige wässerige Lösung, die ebenfalls über saure und basische Ionenaustauscher entsalzt wird.

Auf die Verwendung von organischen Lösungsmitteln, wie Amylalkohol, Ethylacetat oder Diethylether, zur Vorreinigung der wässerigen Phase wird hingewiesen.

l) Auch in neueren Verfahren (SU 1 155 604, zit. in Chem. Abstr. 104, 34 285c (1986)) wird noch zur Reindarstellung von Levoglucosan die Kristallisation aus Aceton und das Umkristallisieren aus 90-96 %igem Ethanol bei 15-30° C beschrieben.

Für eine (groß-)technische Herstellung von Levoglucosan in hoher Reinheit sind die bekannten Verfahren zu umständlich und/oder zu teuer. Als preiswerter Ausgangsstoff ist an sich Stärke sehr gut geeignet.

Die Nachteile der bekannten Stärkepyrolyse (Methods Carbohydr. Chem. 2, 394 (1963)) sind folgende:

1. Die eingesetzte pulverförmige Stärke wird bei 100° C, 24 Stunden lang gründlich unter gelegentlichem Mischen getrocknet. Dies ist umständlich und teuer.

2. Die Pyrolyse in einer Glasapparatur mit einem Gasbrenner führt zu Schichtungen, die den Wärmeübergang negativ beeinflussen, so daß die Masse zwischendurch aufgelockert werden soll, um den Kontakt und dadurch den Wärmeübergang von der Behälterwand zu verbessern.

3. Das Destillat, ein dunkelbrauner Sirup, wird zweimal zur Trokkne mit rel. großen Mengen Aceton unter vermindertem Druck eingedampft, der Rückstand in Aceton aufgenommen und nach Zugabe von Impfkristallen über Nacht bei 0-5° C gehalten. Die rohen dunkelbraunen Kristalle werden abgesaugt, mehrfach mit Aceton gewaschen, getrocknet und in heißem Methanol unter Zugabe von Aktivkohle umkristallisiert, wobei ein technisches Produkt entsteht.

Die endgültige Reinigung erfolgt dann über das Triacetat, das mit Natriummethylat in Methanol entacetyliert, entsalzt und der Feststoff aus Methanol umkristallisiert wird.

Nicht zuletzt wegen dieser umständlichen Verfahren wird in einem Übersichtsartikel in "Adv. in Carbohydr. Chem. Biochem. 39, 157 (1981)" als bestbekannte Synthese von Levoglucosan die alkalische Behandlung von Phenyl-β-D-glucopyranosid bezeichnet. Über die Pyrolyse als Methode zur Synthese von 1,6-Anhydrozuckern wird geschrieben: "Eine Vielzahl von Nebenprodukten und ein Verkohlungsrückstand wird normalerweise gebildet; langwierige Reinigungsverfahren und langsame Kristallisationen sind nicht unüblich. Negative Katalyse, wechselnde Ergebnisse und eine unerfreuliche Ästhetik beeinträchtigen den gelegentlichen Gebrauch dieser Methode...".

Wie bei vielen anderen chemischen Verfahren ist auch die Isolierung des Levoglucosans aus dem Reaktionsgemisch das entscheidende Problem zur Reindarstellung, zumal die Pyrolyse eines Polymers wie der Stärke zu einem breiten Produktspektrum führt.

In Adv. in Carbohydr. Chem. 34, 23 (1977) wird die Zahl von mehr als 37 Verbindungen von niedrigem Molekulargewicht, die also auch flüchtig sind, erwähnt, die bei einer Pyrolyse entstehen. Darunter sind so unterschiedliche Substanzen, wie Furfural, Furan, Acetaldehyd, Aceton, Acrolein, Ameisensäure und Essigsäure.

Nicht zuletzt deshalb gehen neuere Synthesen von Derivaten des Monosaccharids Glucose, z. B. Glycosiden, aus.

Eine methode, aus einem gut zugänglichen nachwachsenden Rohstoff wie Stärke in einer einfachen Reaktion und einer einfachen Isolierung das gewünschte Levoglucosan in guter Ausbeute herzustellen, wäre daher bei der vielseitigen Verwendbarkeit dieses einfachen Glucosederivates ein großer Fortschritt.

Im erfindungsgemäßen Verfahren ist es gelungen,

a) durch eine geeignete Methode der Pyrolyse (Depolymerisation) von Stärken und/oder stärkehaltigem bzw. cellulosehaltigem Material

und

b) durch eine chromatographische Aufarbeitung des Destillates über Ionenaustauscher, einer Methode, die heute im Tonnen-Maßstab industriell genutzt wird

und

c) durch eine Verdampfungs- und/oder Kühlungskristallisation levoglucosanhaltiger Sirupe,

Levoglucosan in guter Ausbeute und hoher Reinheit und mit Wasser als einzigem Lösungsmittel in kristalliner Form herzustellen. Die dabei einzuhaltenden Maßnahmen ergeben sich aus der Anspruchsfassung.

Es kann als überraschend angesehen werden, daß mit derartig einfachen Mitteln Levoglucosan in so hoher Reinheit selbst im großtechnischen Maßstab wirtschaftlich gewonnen werden kann.

Im Zuge des erfindungsgemäßen Verfahrens werden die stärkehaltigen Rohstoffe, z.B. handelsübliche Stärke mit einem durchschnittlichen Wassergehalt bis zu 14% oder Weizenmehl Type 812, in eine Pyrolyseapparatur aus Metall eingebracht, so daß eine Schicht von 1-10 cm den Boden gleichmäßig bedeckt.

Der Wassergehalt kann auf max. 20 % durch Zugabe von Wasser eingestellt werden, da dadurch die Bildung einer gleichmäßig hohen Schicht erleichtert wird.

Die Apparatur (Fig. 1) wird langsam evakuiert, bis ein Druck von 0,0015-6,67 kPa, vorzugsweise 2,00-3,34 kPa, erreicht ist. Dann wird der Boden der Pyrolyseapparatur beheizt, z. B. mit einem Ring-Gas-Brenner mit geeignetem Durchmesser. Ein beispielhaftes Zeit/Temperaturdiagramm ist in Fig. 2 angegeben. Die Temperatur im Kühler wird so geregelt, daß das Kondensat eine Temperatur zwischen 30° und 90° C annimmt und dadurch noch gut fließfähig bleibt. Zwei hintereinander geschaltete Vorlagekolben werden durch Eintauchen in ein Wasserbad zusätzlich gekühlt. Die Endtemperatur im Pyrolysegut sollte vorzugsweise etwa 430 bis 470° C betragen, da dadurch eine leichte Reinigung und Ausräumung des Rückstandes möglich ist.

Das gefärbte wasserhaltige Destillat hat durch die entstandenen flüchtigen Säuren einen pH-Wert von ca. 3 und wird nach Abkühlen und Belüften der Apparatur sofort mit einer Base in fester Form oder als wässerige Lösung auf pH 6 bis 8 gebracht. Die Art der zugesetzten Base richtet sich zweckmäßig nach der Form, in der sich das Ionenaustauscherharz für die sich anschließende Chromatographie befindet. Wird z. B. über eine Ionenaustauschersäule in $Ca^{2+}$-Form chromatographiert, ist es zweckmäßig, das Pyrolysedestillat mit $Ca(OH)_2$ oder $CaCO_3$ zu neutralisieren.

Die Behandlung der filtrierten und/oder neutralisierten Lösung mit einem Adsorptionsmittel, z. B. mit Aktivkohle zum Entfernen hochmolekularer Verunreinigungen, verlängert die Standzeit des Chromatographieharzes.

Ein Vorteil des erfindungsgemäßen Verfahrens ist es, daß die wässerige Lösung bereits in dem für eine präparative Chromotographie günstigen Konzentrationsbereich von 35-70 % TS anfällt, d.h., daß ggf. eher mit Wasser verdünnt als aufkonzentriert werden muß.

Die Chromotographie erfolgt mit Wasser als Elutionsmittel bei einer Temperatur zwischen 20 bis 100° C, vorzugsweise zwischen 30° und 95° C, insbesondere zwischen 50 bis 70° C, und einer linearen Strömungsgeschwindigkeit von 2-8 cm/min, vorzugsweise 3-5 cm/min (vgl. Beispiel 1 Pyrolysate A und B).

Als Chromatographieharze können Kationenaustauscher in einwertiger Salzform (z. B. $Na^{+}$), in zweiwertiger Form (z. B. $Ca^{2+}$) oder in dreiwertiger Form (z. B. $Al^{3+}$) verwendet werden. Die Ionenaustauscher können z. B. sein: stark saure, hochvernetzte, makroporöse Kationenaustauscher auf Polystyrolsulfonsäurebasis (z. B. Lewatit SP 112), schwach saure, makroporöse Kationenaustauscher auf Polyacrylatbasis (z. B. Lewatit CNP LF) oder stark saure, schwachvernetzte, gelartige Kationenaustauscher (z. B. Lewatit TSW 40). Indessen kommen auch andere Kationenaustauscher bei dem erfindungsgemäßen Verfahren in Frage. Vorteilhaft beim erfindungsgemäßen Verfahren ist, daß Farbstoffe und salzartige Verunreinigungen deutlich vor den levoglucosanhaltigen Fraktionen, die durch einen negativen Drehwert am Polarimeter angezeigt werden, eluiert werden.

Erfindungsgemäß kann Levoglucosan durch eine Ionenaustauscherchromatographie ebenfalls rein dargestellt werden aus Reaktionsgemischen, die aus anderen Rohstoffen z. B. Cellulose oder Glucosiden und in anderen Verfahren z. B. basenkatalysierter HF-Eliminierung von α-D-Glucopyranosylfluorid hergestellt wurden und als wässerige Aufgabelösungen der Chromatographie zugeführt werden.

Diese Verfahren können sowohl chargenweise als auch kontinuierlich durchgeführt werden (vgl. Die Stärke 22, 221 (1970)).

Die bei der Chromatographie anfallenden Fraktionen mit einem Levoglucosangehalt von >75% in der Trockensubstanz werden vereinigt und im Vakuum oder im Normaldruckbereich bei einer Temperatur bis 110 °C konzentriert.

In einem Vakuumverdampfungskristallisator wird der eingedickte levoglucosanhaltige Sirup durch Wasserverdampfung leicht übersättigt, z. B. 76 % TS bei 40 °C, Übersättigung ca. 1,06 (vgl. Fig. 3), dann werden Impfkristalle zugegeben und isotherm die Übersättigung unter Rühren abgebaut, wobei es zu einem Kristallzuwachs kommt.

Durch Nachziehen der zu kristallisierenden Lösung unter gleichzeitigem Verdampfen von Wasser bildet sich ein Kristallmagma aus, das nach Beendigung des Kristallisationsvorganges in einer Siebkorbzentrifuge in Kristalle und Mutterlauge getrennt wird.

Alternativ kann auch durch eine Kühlungskristallisation nach Impfen mit Kristallen am Sättigungspunkt und langsamem Abkühlen auf die Temperatur, bei der z. B. eine 50 %ige Kristallausbeute erreicht wird (vgl. Fig. 4) eine Kristallsuspension erhalten werden, die in einer Siebkorbzentrifuge oder einer Nutsche in Kristalle und Mutterlauge aufgetrennt wird.

Auch eine kombinierte Verdampfungs-/Kühlungskristallisation ist möglich, indem zunächst in einer Verdampfungskristallisation bei erhöhter Temperatur (ca. 80 °C) ein Magma erzeugt wird und anschließend die Kristallsuspension unter Bewegung auf Raumtemperatur abgekühlt wird.

Die hohen Reinheiten der bei der erfindungsgemäßen Chromatographie anfallenden levoglucosanhaltigen Lösungen sind der Grund dafür, daß ein grobkörniges, gut zentrifugierbares Kristallisat anfällt, das bereits nach der ersten Kristallisation eine hohe Reinheit aufweist.

## Beispiel 1

### Pyrolyse

3 kg handelsübliche Stärke mit einem Wassergehalt von 14 % wird in eine Metall-Pyrolyseapparatur so eingebracht, daß eine gleichmäßige Schichtdicke (ca. 8 cm) den leicht konkav gewölbten Boden bedeckt. Es besteht die Möglichkeit, im Pyrolysegut sowie im Destillatübergang die Temperatur zu messen. Der Kühler besteht ebenfalls aus Metall. Mit einem geeigneten Ring-Gas-Brenner wird das Pyrolysegut erhitzt, nachdem die gesamte Apparatur mit einer Dampfstrahlpumpe auf einen Druck von ca. 3,34 kPa evakuiert wurde. Ein Zeit/Temperaturverhältnis ist in Fig. 2 beispielhaft dargestellt.

Das Ende der Reaktion ist erreicht, wenn im Gut die Temperatur 450-470 °C beträgt und im Übergang die Temperatur zurückgeht.

Nach Abkühlen und Belüften der Apparatur wird mit wenig Dampf der Kühler gereinigt, um Spuren des sirupösen Destillats in die Vorlage zu überführen.

Das Pyrolysat (ca. 2,1 kg) hat einen pH-Wert von etwa 3 und wird mit Calciumhydroxid (ca. 60 g) auf pH 6,5 gebracht, über ein Filterhilfsmittel, z. B. Kieselgur, filtriert und anschliessend chromatographiert.

Aus 3,0 kg Stärke erhält man 2,74 kg filtriertes und neutralisiertes Destillat mit einem refraktometrisch gemessenen Brechungsindex von 1,444 ( ≙ 61 TS-Gehalt) und einem Levoglucosangehalt von 59 %, bezogen auf den TS-Gehalt und über HPLC bestimmt (Säulenlänge 30 cm, Trennmittel BIO-RAD HPX-87C, Elutionsmittel Wasser, Temperatur 85 °C, Flußgeschwindigkeit 0,5 ml/min).

Die Konzentrationsbestimmung für Levoglucosan in wässerigen Lösungen erfolgt refraktometrisch nach Tabellen aus Latv. PSR Zinat. Akad. Vestis Kim. Ser. 1967 (1), 119-221 (zit. in Chem. Abstr. 67, 50 437 b).

### Chromatographie von Stärkepyrolysaten A

Die Destillate mehrerer Pyrolyseansätze werden, nach Behandlung mit Aktivkohle, in Form einer 47%igen wässerigen, auf 65 °C erwärmten Lösung (18,5 kg) als Aufgabelösung einer Chromatographieanlage zugeführt; diese enthält sulfoniertes divinylbenzolvernetztes Polystyrolharz in der $Ca^{2+}$-Form, der Ionenaustauscher ist stark sauer und gelförmig. Die Anlage besteht aus drei Säulen, Innendurchmesser 25 cm, Gesamtinhalt 500 l.

Mit vollentsalztem Wasser wird bei 65 °C eluiert, ein Teilstrom des in Reihe aus der dritten Säule kommenden Elutionsstroms wird durch ein Refraktometer, Polarimeter und Leitfähigkeitsmeßgerät geleitet

und die Meßwerte registriert. Es ergibt sich das in Fig. 5 dargestellte Diagramm.

Die Extinktion wurde nachträglich bei 420 nm in einzelnen Fraktionen bestimmt. Die Extinktion bei 420 nm ist ein Maß für die Farbe der Lösung.

Wie man sieht, lassen sich salzartige (Leitfähigkeit) und farbige Verunreinigungen vor der Levoglucosanfraktion, die durch negative Drehwerte am Polarimeter erkennbar ist, gut abtrennen.

Die Aufgabelösung enthielt 63,2 % Levoglucosan und 36,8 % Verunreinigungen, bezogen auf den TS-Gehalt. Die Einzelfraktionen 1-14 (2,42 kg refraktometrisch berechnet) werden verworfen, die Fraktionen 15 und 16 werden vereinigt und rechromatographiert und die Produktfraktion (Einzelfraktionen 17-23, 4,82 kg refraktometrisch bestimmt) wird eingeengt und einer Verdampfungskristallisation unterworfen.

Chromatographie von Stärkepyrolysaten B

Die Zusammensetzung einer Chromatographie analog der Chromatographie A ist in Fig. 6 dargestellt. Die Aufgabelösung enthielt 63,8 % Levoglucosan, Gesamtmenge 11,5 kg TS als 30 %ige wässerige Lösung. Die Fraktionen I-VI enthalten:

| | | | | |
|---|---|---|---|---|
| Fraktion I | 0 % | Levoglucosan | mit $n_D^{20}$ | = 1,3358 |
| Fraktion II | 14,5 % | Levoglucosan | " " | = 1,3375 |
| Fraktion III | 75,8 % | Levoglucosan | " " | = 1,3423 |
| Fraktion IV | 93,4 % | Levoglucosan | " " | = 1,3417 |
| Fraktion V | 96,6 % | Levoglucosan | " " | = 1,3400 |
| Fraktion VI | 90,6 % | Levoglucosan | " " | = 1,3352 |

Die Fraktion I wird verworfen, die Fraktion II wird rechromatographiert und die Fraktionen III-VI werden vereinigt, eingedampft und einer Verdampfungskristallisation unterworfen.

Kristallisation

Die bei der chromatographischen Trennung anfallenden Fraktionen III-V (s. Beispiel oben) werden in einem Dünnschichtverdampfer auf einen TS-Gehalt von ca. 50 % konzentriert (14,2 kg mit $n_D^{20}$ =1,4173).

In einem Rotationsverdampfer wird ein Teil im Vakuum auf eine Konzentration von 80 % weiter eingeengt, mit Levoglucosankristallen bei 45° C geimpft, bis zu einem sichtbaren Kristallwachstum weiter Wasser verdampft (ca. 10 min) und die Hauptmenge der vereinigten Fraktionen III-V langsam nachgezogen. Anschließend wird unter Bewegung die Kristallsuspension langsam auf Raumtemperatur (25° C) abgekühlt und in einer Siebkorbzentrifuge in Kristalle (3,28 kg, Reinheit 98,8 %) und Mutterlauge (3,65 kg) aufgetrennt.

Die Mutterlauge wird zusammen mit der Fraktion VI einer zweiten Kristallisation (wie oben) unterworfen. Man erhält 2,17 kg Kristalle mit einer Reinheit von 99, 0 % sowie eine Mutterlauge von 3,76 kg mit einem Brechungsindex von 1,4514. Die Mutterlauge wird rechromatographiert. In der Ausgangslösung waren 7,23 kg Levoglucosan. Die Gesamtkristallausbeute an Levoglucosan beträgt somit 5,45 kg (75,4 %) mit einer Reinheit > 98,8 % und einem Schmelzpunkt von 175° C.

Beispiel 2

Chromatographie eines Weizenmehlpyrolysedestillats

847 g mit einem Brechungsindex von 1,3776 entsprechend ca. 240g TS eines Destillates, das bei der Pyrolyse von Weizenmehl Typ 812 und nach Aktivkohlebehandlung anfällt (Levoglucosangehalt mit HPLC zu 41, 6 %, bez. auf TS bestimmt), wird über eine Säule (Länge 200 cm, Innendurchmesser 8 cm, gefüllt mit Lewatit TSW 40 in der $CA^{2+}$-Form) mit Wasser als Elutionsmittel bei 70° C chromatographiert.

Das Chromatogramm hat Ähnlichkeit mit der Fig. 5. Fraktioniert wird ab dem im Eluat gemessenen Brechungsindex $n_D^{20}$ =1,335, die Fraktionsgröße ist 0,5 l; die Fraktion 9 (73,8 % Levoglucosan), Fraktion 10 (79,8 % Levoglucosan), Fraktion 11 (87,8 % Levoglucosan) und Fraktion 12 (74,3 % Levoglucosan jeweils bezogen auf TS) werden vereinigt und zur Kristallisation gebracht.

Eine einfache und schnelle Methode hochangereicherte levoglucosanhaltige Fraktionen zu ermitteln, ist die Dünnschichtchromatographie auf Kieselgel 60 $WF_{254}$ s, Laufmittel $CH_3CN/H_2O$; 80/20, Detektion: Veraschen, $R_f$-Wert: 0,48.

## Ansprüche

1. Verfahren zur Herstellung von 1,6-β-D-Anhydroglucopyranose (Levoglucosan) in hoher Reinheit aus Stärke und/oder stärke- haltigem bzw. cellulosehaltigem Material durch Pyrolyse, dadurch gekennzeichnet, daß das Ausgangsmaterial mit einem auf max. 20 % eingestellten Wassergehalt im Vakuum pyrolysiert und die flüchtigen Bestandteile kondensiert werden, dieses Kondensat nach Neutralisation, Filtration und ggf. Behandlung mit einem Adsorptionsmittel einer chromatographischen Trennung über Ionenaustauscher mit Wasser als Elutionsmittel unterworfen wird, die Levoglucosan enthaltenden Fraktionen eingeengt werden und dann aus den wässerigen Lösungen durch eine Verdampfungs- und/oder Kühlungskristallisation kristallines Levoglucosan gewonnen wird.

2. Verfahren nach Anspruch 1, dadurch gekennzeichnet, daß pulverförmige Stärke mit einem handelsüblichen Wassergehalt von 1o - 18 % auf einen Wassergehalt von max. 2o % gebracht wird.

3. Verfahren nach den Ansprüchen 1 und 2, dadurch gekennzeichnet, daß das stärkehaltige Material in einer Schicht von 1 bis 1o cm Dicke in einem evakuierbaren Gefäß pyrolysiert wird und hierbei ein Vakuum zwischen 0,0013 und 13,34 kPa, vorzugsweise zwischen 2,00 und 3,34 kPa angelegt wird.

4. Verfahren nach den Ansprüchen 1 bis 3, dadurch gekennzeichnet, daß eine Temperatur in der zu pyrolysierenden Masse zwischen 150° C und 500° C, vorzugsweise zwischen 200° C und 470° C eingehalten wird.

5. Verfahren nach den Ansprüchen 1 bis 4, dadurch gekennzeichnet, daß das Kondensat nach einer pH-Werteinstellung auf 6 bis 8, Filtration und ggf. Entfernung hochmolekularer Stoffe mit einem Adsorptionsmittel, wie Aktivkohle, der nachfolgenden chromatographischen Trennung mit Wasser als Elutionsmittel, ggf. nach Verdünnung der Aufgabelösung, unterworfen wird.

6. Verfahren nach den Ansprüchen 1 bis 5, dadurch gekennzeichnet, daß das aufbereitete und filtrierte Pyrolysedestillat an zur chromatographischen Trennung geeigneten Kationenaustauscherharzen, die in ein-, zwei- oder drei-wertiger Salzform vorliegen, chromatographiert wird, wobei das Kation der Harze mit dem Kation der Base, die zum Neutralisieren verwendet wurde, übereinstimmt.

7. Verfahren nach den Ansprüchen 1 bis 6, dadurch gekennzeichnet, daß Ionenaustauscher auf der Basis von stark sauren, hochvernetzten, makroporösen Kationenaustauscher auf Polystyrolsulfonsäurebasis oder schwach sauren, makroporösen Kationenaustauscher auf Polyacrylatbasis oder stark sauren, schwachvernetzten, gelartigen Kationenaustauschern, zum Einsatz kommen.

8. Verfahren nach den Ansprüchen 1 bis 7, dadurch gekennzeichnet, daß die Chromatographie mit Wasser unter sonst bekannter üblicher linearer Strömungsgeschwindigkeit und Aufgabenmenge bei einer Temperatur zwischen 20 und 100° C, vorzugsweise 50 - 70° C, durchgeführt wird.

9. Verfahren nach den Ansprüchen 1 bis 8, dadurch gekennzeichnet, daß die Fraktionen, die Levoglucosan enthalten, ggf. im Vakuum, eingeengt werden bis zu dem Trockensubstanzgehalt, der einer Übersättigung von 1,01-1,10 entspricht, und durch Kühlung oder Wasserverdampfung das darin enthaltene Levoglucosan zur Kristallisation gebracht werden.

10. Verfahren nach den Ansprüchen 1 bis 9, dadurch gekennzeichnet, daß Mutterlaugen aus der Kristallisation allein oder vereinigt mit chromatographischen Fraktionen, die weniger als 75 % Levoglucosan in der Trockensubstanz enthalten, erneut einer Chromatographie nach Anspruch 1 unterworfen werden.

11. Anwendung der in den Ansprüchen 1 bis 10 angeführten Maßnahmen zur Chromatographischen Trennung über Ionenaustauscher mit Wasser als Elutionsmittel sowie Einengung der Fraktionen und Gewinnung von kristallinem Levoglucosan auf nicht durch Pyrolyse hergestellte wässerige levoglucosanhaltige Lösungen.

FIG. 1

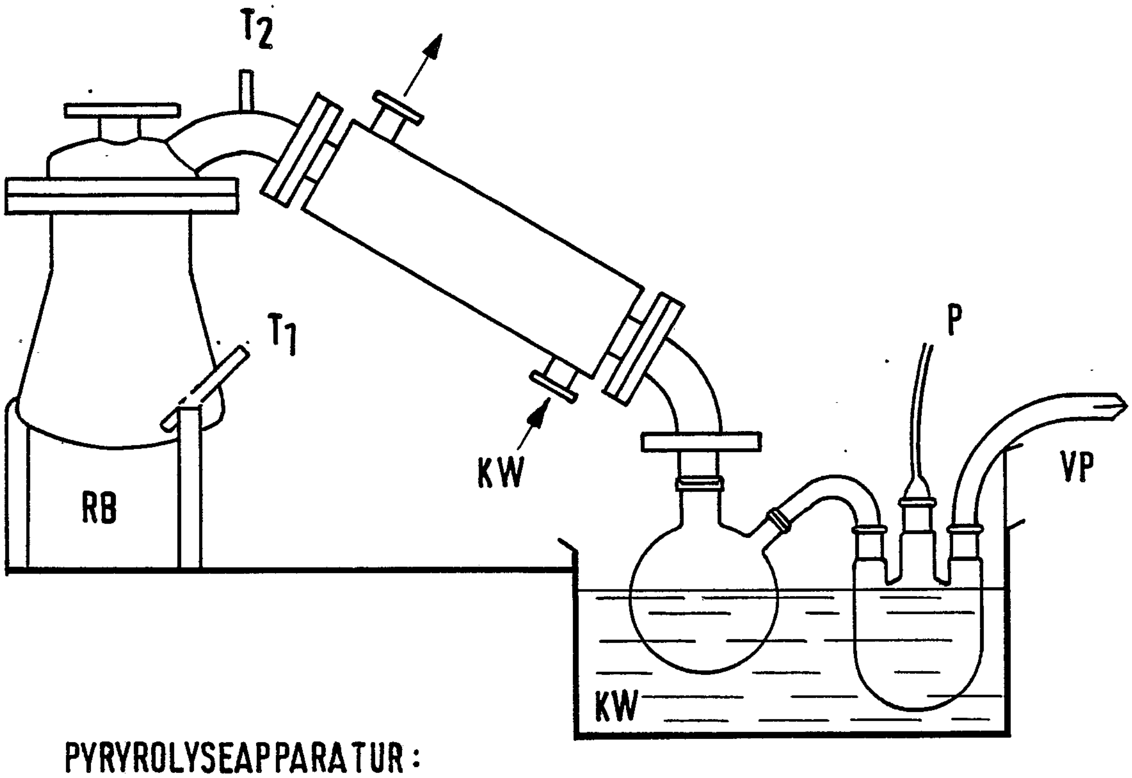


PYRYROLYSEAPPARATUR:

RB = RINGBRENNER

$T_1$, $T_2$ = TEMPERATURMESSSTELLEN

KW = KÜHLWASSER

P = DRUCKMESSUNG

VP = VAKUUMPUMPE

FIG. 2

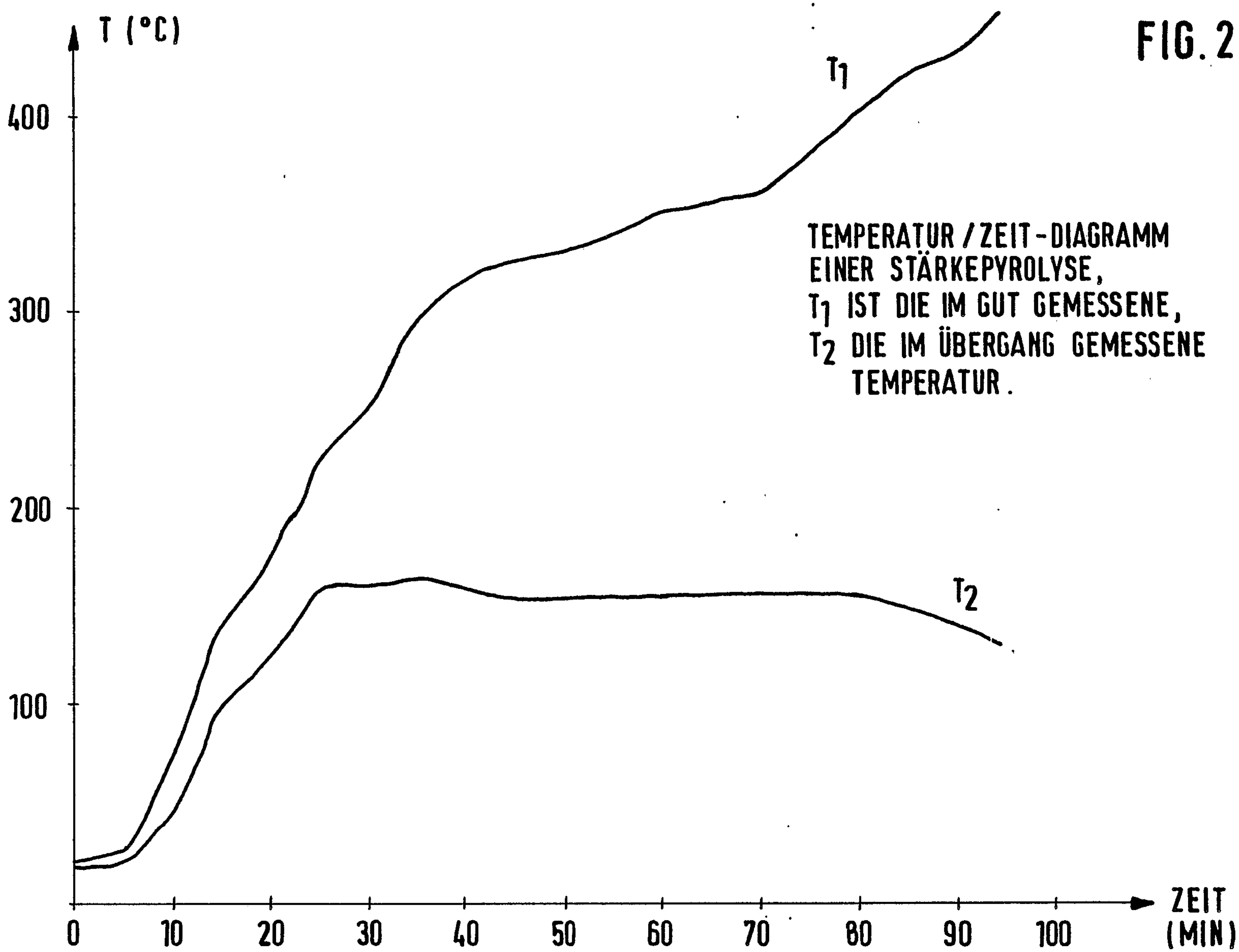
T (°C)
400
300
200
100
$T_1$
$T_2$
TEMPERATUR / ZEIT-DIAGRAMM
EINER STÄRKEPYROLYSE,
$T_1$ IST DIE IM GUT GEMESSENE,
$T_2$ DIE IM ÜBERGANG GEMESSENE
TEMPERATUR.
0
10
20
30
40
50
60
70
80
90
100
ZEIT
(MIN)

FIG. 3

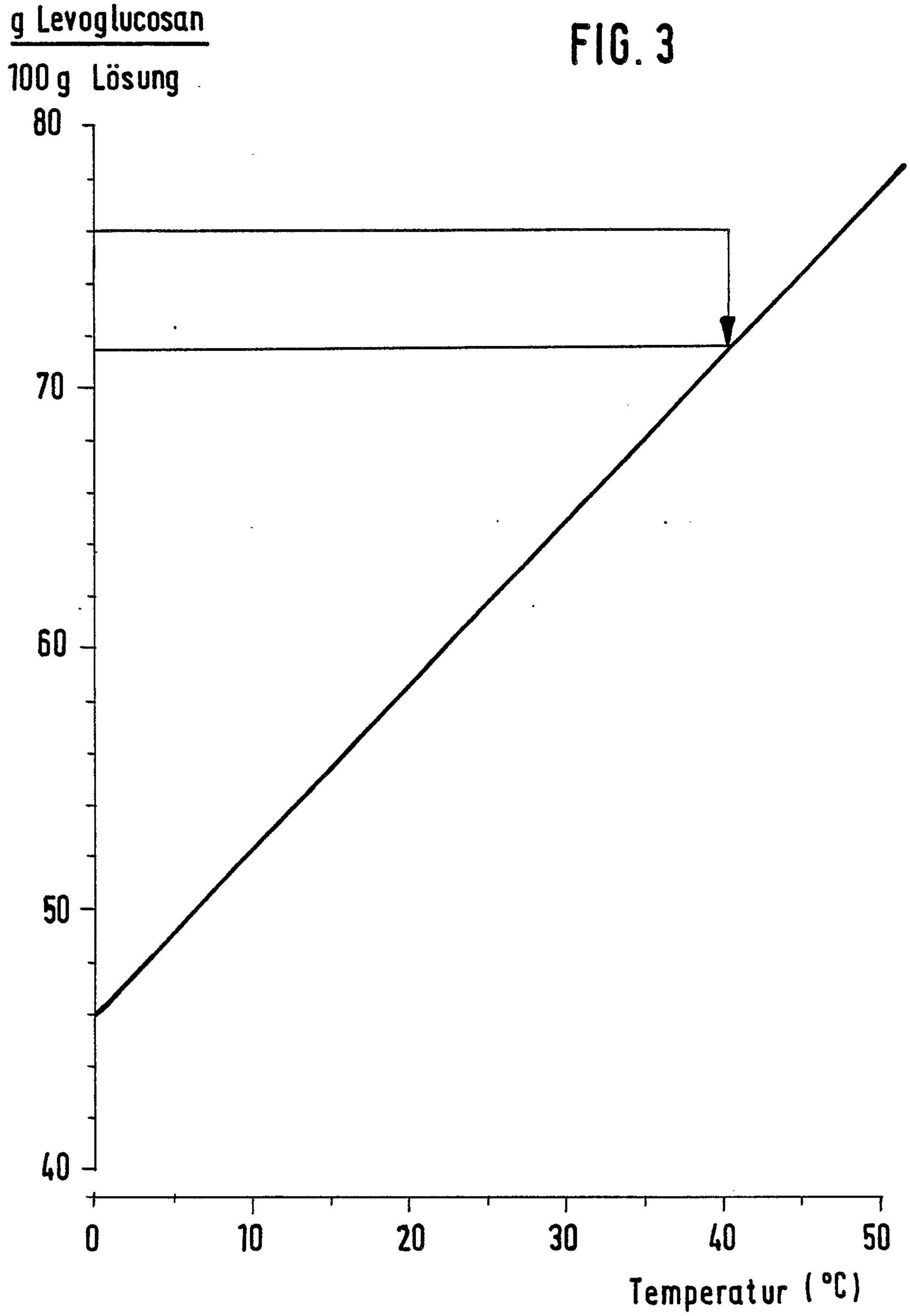
g Levoglucosan
100 g Lösung
80
70
60
50
40
0
10
20
30
40
50
Temperatur (°C)

FIG. 4

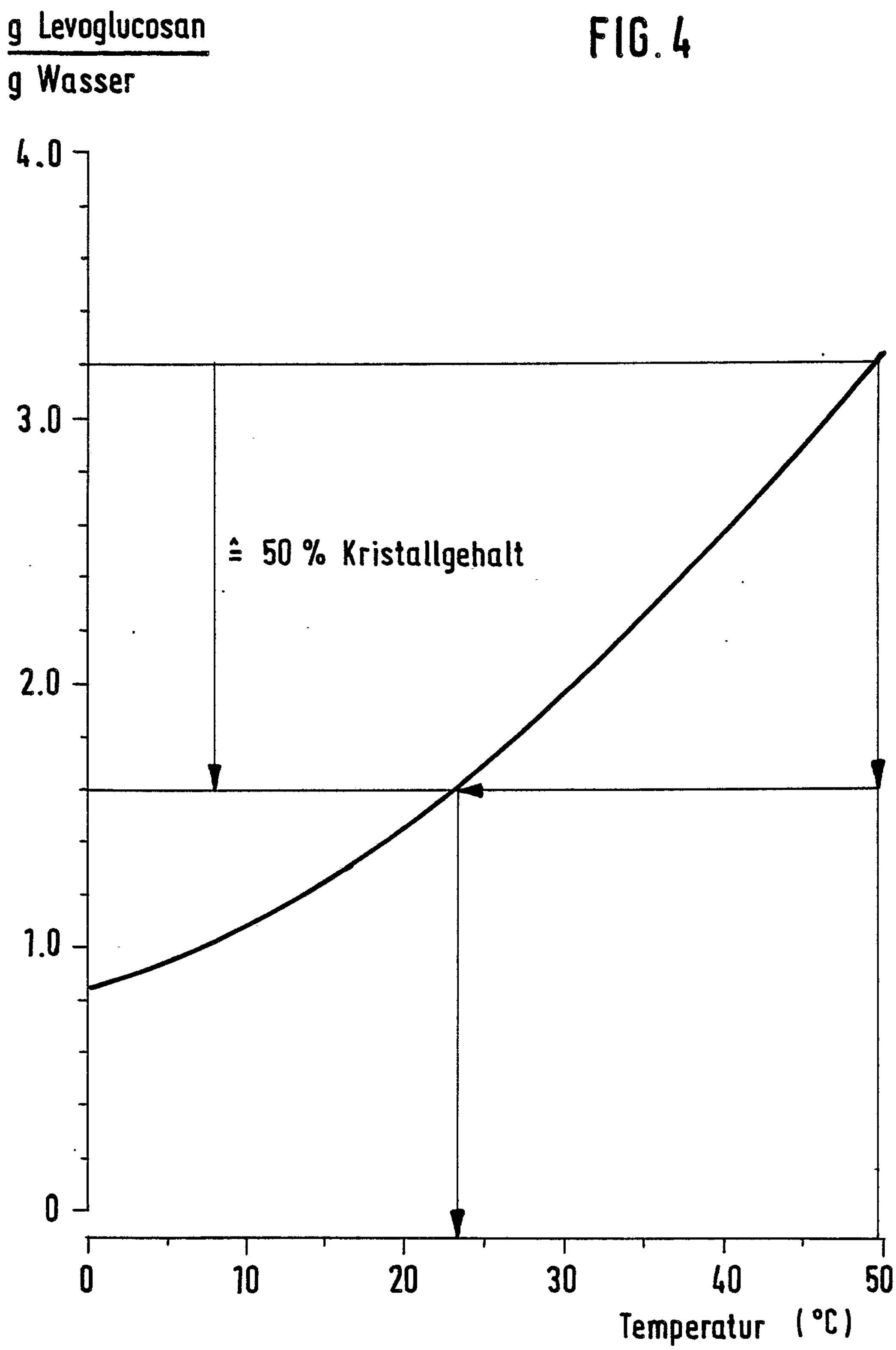
g Levoglucosan / g Wasser
4.0
3.0
2.0
1.0
0
≙ 50 % Kristallgehalt
0
10
20
30
40
50
Temperatur (°C)

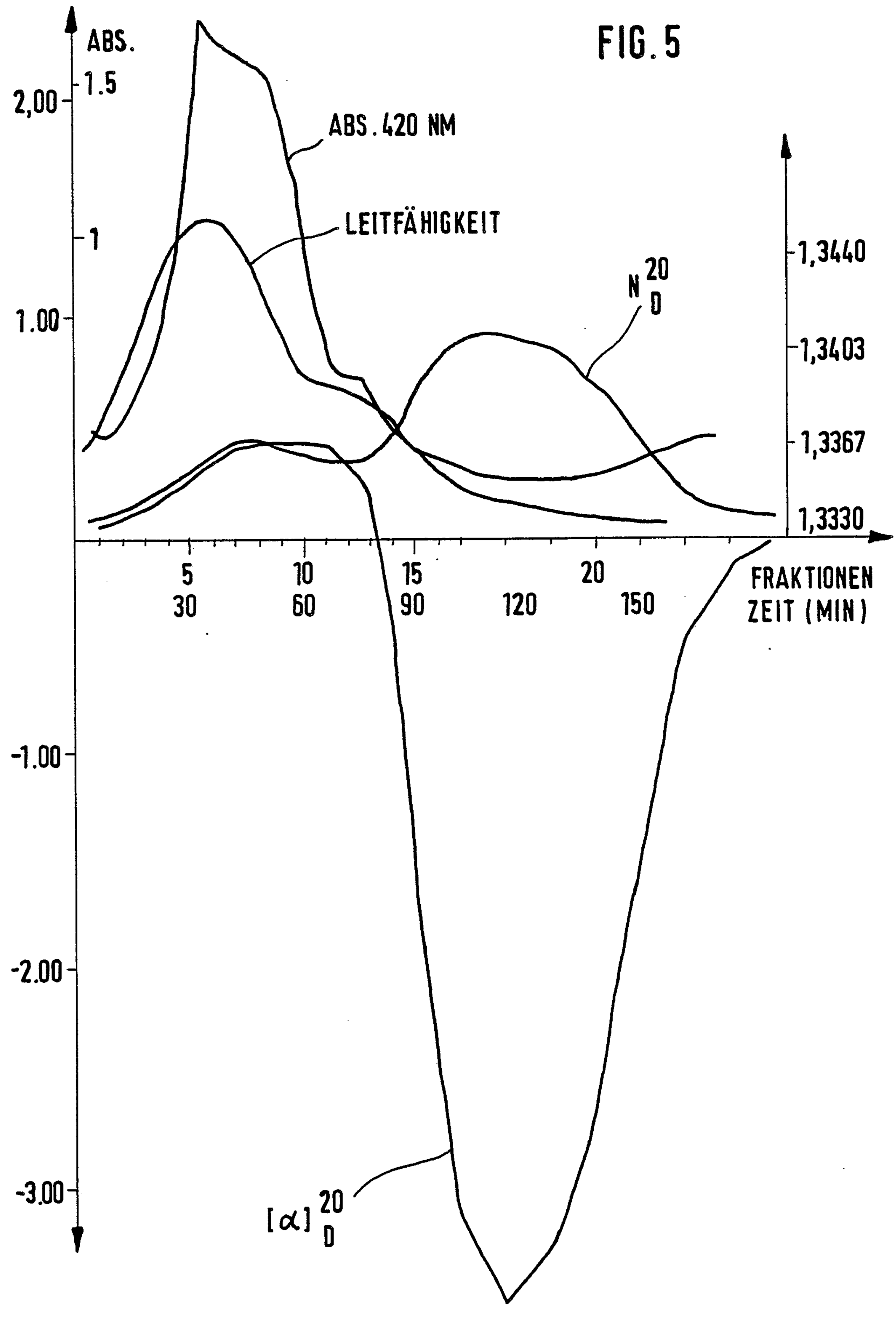
ABS.
1.5
1
2,00
1.00
-1.00
-2.00
-3.00
ABS. 420 NM
LEITFÄHIGKEIT
N 20 D
1,3440
1,3403
1,3367
1,3330
5
10
15
20
30
60
90
120
150
FRAKTIONEN
ZEIT (MIN)
[α] 20 D

FIG. 5

FIG.6

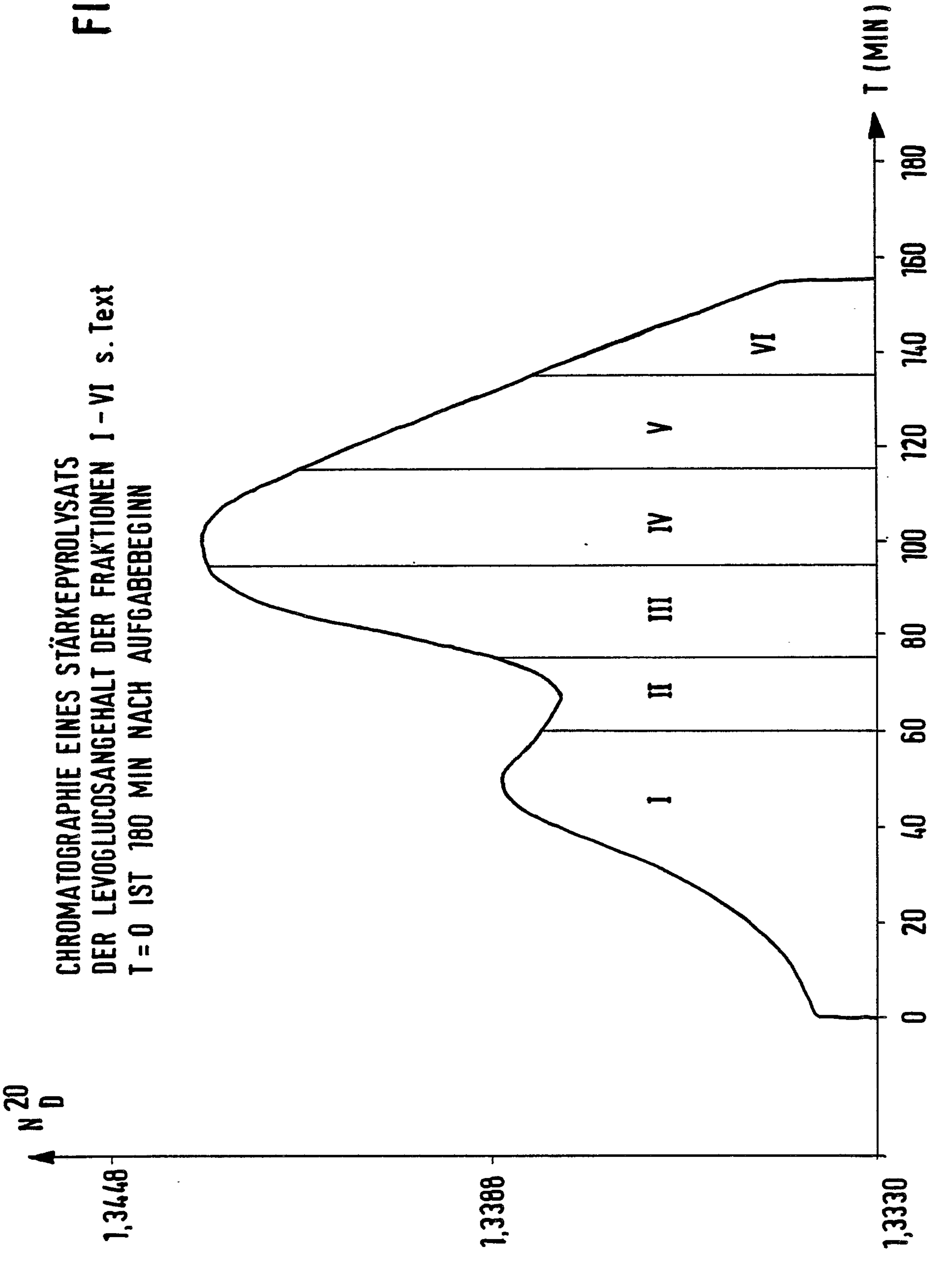
CHROMATOGRAPHIE EINES STÄRKEPYROLYSATS
DER LEVOGLUCOSANGEHALT DER FRAKTIONEN I - VI s. Text
T = 0 IST 180 MIN NACH AUFGABEBEGINN
$N_D^{20}$
1,3448
1,3388
1,3330
T (MIN)
0
20
40
60
80
100
120
140
160
180
I
II
III
IV
V
VI